(19) Europäisches Patentamt

European Patent Office

Office européen des brevets

(11) **EP 1 506 950 A1**

(12) **EUROPEAN PATENT APPLICATION**

(43) Date of publication:
**16.02.2005 Bulletin 2005/07**

(51) Int Cl.⁷: **C07C 37/70**, C07C 39/235,
C07C 51/15, C07C 65/11

(21) Application number: **04019324.5**

(22) Date of filing: **13.08.2004**

(84) Designated Contracting States:
**AT BE BG CH CY CZ DE DK EE ES FI FR GB GR
HU IE IT LI LU MC NL PL PT RO SE SI SK TR**
Designated Extension States:
**AL HR LT LV MK**

(30) Priority: **13.08.2003 JP 2003293058**

(71) Applicant: **KABUSHIKI KAISHA UENO SEIYAKU
OYO KENKYUJO
Osaka-shi, Osaka-fu (JP)**

(72) Inventors:
• **Ueno, Ryuzo**
**Nishinomiya-shi Hyogo-ken (JP)**
• **Kitayama, Masaya**
**Takarazuka-shi Hyogo-ken (JP)**
• **Izumichi, Nobutaka**
**Ashiya-shi Hyogo-ken (JP)**
• **Obata, Akira**
**Nishinomiya-shi Hyogo-ken (JP)**

(74) Representative: **Albrecht, Thomas, Dr. et al
Kraus & Weisert,
Thomas-Wimmer-Ring 15
80539 München (DE)**

(54) **Process for dehydrating hydrous sodium Beta-naphtholate**

(57)    The present invention provides a process for dehydrating hydrous sodium β-naphtholate comprising: the step of heating the hydrous sodium β-naphtholate or a mixture of the hydrous sodium β-naphtholate and β-naphthol which comprises no more than 0.2 mole of β-naphthol per one mole of sodium β-naphtholate in a solvent at a temperature of 260-300°C under an inert gas. The process makes it possible to dehydrate hydrous sodium β-naphtholate thoroughly in a short time with less production of tarry components.

**Description**

FIELD OF THE INVENTION

The present invention relates to a process for dehydrating sodium β-naphtholate. The invention also relates to a process for preparing 2-hydroxy-3-naphthoic acid comprising the dehydrating step.

2-hydroxy-3-naphthoic acid is a useful intermediate for producing pigments, dyes and the like.

Conventional process for preparing 2-hydroxy-3-naphthoic acid comprises reacting β-naphthol with sodium hydroxide to give sodium β-naphtholate, dehydrating the sodium β-naphtholate thoroughly and then, reacting with carbon dioxide to give sodium 2-hydroxy-3-naphtholate, dissolving the sodium 2-hydroxy-3-naphtholate in water and isolating 2-hydroxy-3-naphthoic acid by means of acid crystallization.

A solid-gas phase reaction, known as Kolbe-Schmitt reaction, has conventionally been used to react a sodium β-naphtholate with carbon dioxide. In the Kolbe-Schmitt reaction, water existing in the system inhibits the reaction and reduces the yield. Accordingly, the starting material, i.e., alkali metal salts of aromatic hydroxy compounds such as sodium β-naphtholate must be sufficiently dehydrated.

The dehydration of hydrous sodium β-naphtholate in solid phase takes a pretty long time and it is difficult to remove the water contained in the crystal. In addition, solid-gas phase Kolbe-Schmitt reaction requires long reaction time, no less than 50 hours. Furthermore, losses of β-naphthol are great because of the thermal nonuniformity of the reaction at high temperatures. The reaction is difficult to control owing to changes in phase during the reaction, and stable yields are difficult to attain.

In order to overcome these difficulties of the Kolbe-Schmitt reaction in solid-gas phase, the process that comprises dehydrating sodium β-naphtholate in liquid phase and then reacting the dehydrated sodium β-naphtholate with carbon dioxide has been proposed. Specifically, the process for dehydrating sodium β-naphtholate that comprises dehydrating the mixture of 2-hydroxy naphthalene (i.e. β-naphthol) and small amount of alkali hydroxide at about 130-200°C under reduced pressure has been proposed. See, for example, Japanese Application Laid Open No. 51-4152 (GB1507054 A), the content of which is herein incorporated by reference.

In the above-described process, large amount of unreacted β-naphthol exists in the reaction system, because the mixture contains β-naphthol and small amount of sodium hydroxide. As a result, it is possible to dehydrate sodium β-naphtholate in liquid phase. However, dehydrating sodium β-naphtholate as a mixture with β-naphthol inevitably causes the generation of the tarry component, which is resin-like side-product, due to the existence of β-naphthol. As a result, the yield of target product, 2-hydroxy-3-naphthoic acid, is decreased.

Moreover, the large amount of β-naphthol lowers the melting point of the eutectic mixture of β-naphthol and sodium β-naphtholate. As a result; the dehydrating temperature of the mixture should be lowered to about 130-200°C and therefore, the dehydrating process takes a long time period.

On the other hand, a process for manufacturing hydroxynaphthalene carboxylic acid which comprises the steps of dehydrating sodium β-naphtholate or mixture of sodium β-naphtholate and β-naphthol in light oil or kerosene, and reacting the dehydrated sodium β-naphtholate in liquid form with carbon dioxide has been proposed. See, for example, US 4239913, the content of which is herein incorporated by reference.

In this process, sodium β-naphtholate, alone or in combination with β-naphthol, is dehydrated in solvent. However, the dehydrating temperature is relatively low, about 250°C, and therefore, the dehydrating process requires long time period.

In addition, when dehydrating solid-state sodium β-naphtholate dispersed in solvent at a conventionally used temperature (i.e., no more than 250°C), it is difficult to remove the water contained in the crystalline sodium β-naphtholate. In this process, the dehydrated solid-state sodium β-naphtholate is obtained as dispersion in the solvent or slurry. It is difficult to transfer the dispersion or slurry of dehydrated sodium β-naphtholate to the next step and the hard sodium β-naphtholate crystalline particles may cause galling of the reaction container. Accordingly, the process is not preferable for industrial use.

Therefore, in the conventional process for manufacturing 2-hydroxy-3-naphthoic acid in industrial scale, hydrous sodium β-naphtholate is generally dehydrated as a mixture with β-naphthol. However, the dehydration of the mixture of sodium β-naphtholate and β-naphthol inevitably causes the generation of tarry component due to the existence of β-naphthol.

There is a strong desire for a process for dehydrating the starting material, sodium β-naphtholate, sufficiently in a short time and that for producing 2-hydroxy-3-naphthoic acid in large scale, with high yield at low cost and less side-product such as a tarry component.

SUMMARY OF THE INVENTION

An object of the present invention is providing a process for dehydrating or drying hydrous sodium β-naphtholate which allows the sufficient dryness in a short time with no or less tarry by-product.

Another object of the present invention is providing a process for producing 2-hydroxy-3-naphthoic acid in industrial scale with high yield at low cost.

The present invention provides a process for dehydrating hydrous sodium β-naphtholate comprising:

the step of heating the hydrous sodium β-naphtholate or a mixture of the hydrous sodium β-naphtholate and β-naphthol which comprises no more than 0.2 mole of β-naphthol per one mole of sodium β-naphtholate in a solvent at a temperature of 260-300°C under an inert gas.

The present invention also provides a process for manufacturing 2-hydroxy-3-naphthoic acid, comprising the steps of:

heating the hydrous sodium β-naphtholate or a mixture of the hydrous sodium β-naphtholate and β-naphthol which comprises no more than 0.2 mole of β-naphthol per one mole of sodium β-naphtholate in a solvent at a temperature of 260-300°C under an inert gas to dehydrate the hydrous sodium β-naphtholate; and
reacting the dehydrated sodium β-naphtholate in the solvent with carbon dioxide.

The process of the present invention comprises dehydrating molten-state hydrous sodium β-naphtholate in a solvent such as light oil and white oil and reacting the dehydrated sodium β-naphtholate with carbon dioxide. The process significantly reduces the side-product generated in the dehydration and reaction steps than those in the conventional processes. In addition, the 2-hydroxy-3-naphthoic acid obtained by the process of the present invention has good quality with less coloring due to the presence of tar-like substances.

DETAILED DESCRIPTION OF THE PREFERRED EMBODIMENTS

In the specification and claims, the term "dehydrate" or "dehydration" represents to remove water from the material to be dehydrated; and the term "hydrous" indicates the presence of water in the material.

In the process of the present invention, sodium β-naphtholate to be dehydrated may be contaminated with no more than 0.2, preferably no more than 0.1 mole of β-naphthol per one mole of sodium β-naphtholate. More preferably, sodium β-naphtholate does not contain β-naphthol at all. However, in the industrially used process of producing 2-hydroxy-3-naphthoic acid, sodium β-naphtholate is recycled by isolating it from the end product and the recycled material may contain β-naphthol.

When the amount of β-naphthol is more than 0.2 mole per one mole of sodium β-naphtholate, a tarry component is inevitably generated in the dehydration step and therefore, the yield of 2-hydroxy-3-naphthoic acid is decreased.

In the process of the present invention, the temperature during the dehydration step is no less than 260°C, preferably no less than 270°C and more preferably no less than 280°C. Sodium β-naphtholate is in the molten state at such a high temperature and therefore, is easily dispersed in the solvent. As a result, stirring the mixture and transferring the dehydrated sodium β-naphtholate to the next step is facilitated. Furthermore, due to the high temperature, the industrially advantageous liquid phase dehydration of sodium β-naphtholate can be completed in a short time.

When the mixture of sodium β-naphtholate and β-naphthol is dehydrated at a temperature lower than the eutectic point of the mixture, the mixture may become slurry that contains solid state material.

In the process of the present invention, the temperature during the dehydration step is no more than 300°C. Though it is possible to dehydrate the mixture of sodium β-naphtholate and β-naphthol at a temperature higher than 300°C, there is no advantage of employing such a high temperature and excess heat may cause a waste of energy. Moreover, when the mixture is dehydrated at a temperature higher than 300°C, tar-like substances may be produced due to the exposure of the mixture to such a high temperature.

The hydrous sodium β-naphtholate to be dehydrated by the process of the present invention may be that obtained by reacting a basic sodium compound, such as sodium hydroxide and sodium carbonate, with 0.9-1.1 mole of β-naphthol per one mole of the basic sodium compound under inert gas atmosphere.

It is preferable to react sodium hydroxide with 0.9-1.1 mole of β-naphthol per one mole of sodium hydroxide in water so that an aqueous sodium β-naphtholate solution comprising 40-60 % by weight of sodium β-naphtholate is obtained.

The resulting aqueous sodium β-naphtholate is heated under inert gas flow to concentrate the same without generating solid-state material. To the concentrated hydrous sodium β-naphtholate, 0.5 to 10 parts by weight of solvent per 1 part by weight of sodium β-naphtholate is added.

The solvent may be added at the start of the heating, but preferably it may be added when the concentrating step is completed.

The suitable solvent used for the process of the present invention is light oil, kerosene, white oil or a mixture thereof. Most preferably, light oil alone is used as the solvent. Preferably, solvents are those obtained by distilling off the components having boiling point no more than 260°C, more preferably, no more than 300°C.

Sodium β-naphtholate added with the solvent is heated to no less than 260°C, preferably no less than 270 °C and more preferably no less than 280°C so that it becomes molten state, and is dehydrated under an inert gas with stirring. Non-limiting examples of inert gases include nitrogen gas.

The dehydration step is preferably performed for equal to or less than 2 hours, more preferably 0.5-1.5 hours from the time when the temperature is achieved to the desired dehydration temperature. The 2 hours or less dehydration time will be enough to sufficiently dehydrate the hydrous sodium β-naphtholate and therefore, the dehydration time longer than 2 hours is not recommended because it may cause unnecessary energy consumption.

In the process of the present invention for preparing 2-hydroxy-3-naphthoic acid, the liquid mixture comprising the dehydrated sodium β-naphtholate in the solvent is subjected to the next step, i.e.,reaction with carbon dioxide.

In reacting with carbon dioxide, the amount of the solvent is preferably 0.5-3 parts by weight, more preferably 1-2 parts by weight per 1 part by weight of sodium β-naphtholate.

In reacting sodium β-naphtholate with carbon dioxide, in order to accelerate the reaction, β-naphthol is preferably added to the reaction. The amount of β-naphthol is preferably 0.1-3 moles, more preferably 0.2-0.7 mole per 1 mole of sodium β-naphtholate.

The reaction temperature of sodium β-naphtholate with carbon dioxide is preferably 200-350°C, and more preferably 250-300°C.

During the above reaction, the pressure of carbon dioxide is preferably 0.05-2 MPa (G), and more preferably 0.2-1 MPa (G).

After the reaction is completed, the reaction is cooled, added with water and stirred so that the reaction product is dissolved. Alternatively, the resultant reaction may be transferred to a container containing water using a pump and thereafter the product may be dissolved by stirring.

Thus obtained solution is separated into a solvent layer and a 2-hydroxy-3-naphtholate containing water layer with keeping the temperature of 80-100°C in order to prevent crystallization of sodium 2-hydroxy-3-naphtholate and β-naphthol.

Next, an acid such as an inorganic acid is added to the water layer so that the water layer is adjusted to pH 6.5-8, and then β-naphthol originating from the unreacted sodium β-naphtholate is extracted with a hydrophobic organic solvent.

Examples of suitable hydrophobic organic solvents used for the extraction include; aromatic hydrocarbons such as toluene, xylene and nitrobenzene; alcohols such as n-butanol, n-octyl alcohol and 2-ethylhexylalcohol; ethers such as dibutyl ether and diphenyl ether; and ketones such as cyclohexanone, methylethylketone and methylisobutylketone.

β-naphthol extracted in the solvent layer and in the hydrophobic organic solvent may be dissolved in aqueous sodium hydroxide, and recovered as sodium β-naphtholate. Thus obtained sodium β-naphtholate can be dehydrated according to the invention and used again for the reaction.

The water layer after the β-naphthol is removed is subjected to the acid crystallization. Before the acid crystallization, if necessary, the water layer may be treated with an absorbing agent such as active charcoal in order to remove impurity such as tar-like substances. Non-limiting examples of acids used for the acid crystallization include inorganic acids such as binary acids such as hydrochloric acid and hydrofluoric acid; and oxo acids such as sulfuric acid, nitric acid, phosphoric acid and perchloric acid. The water layer is subjected to acid crystallization using these inorganic acids which adjust the pH of the layer to pH 1-3.

The 2-hydroxy-3-naphthoic acid obtained as suspension by the acid crystallization is separated from the mother liquid by a conventional means such as centrifugation or filter press. If necessary, 2-hydroxy-3-naphthoic acid is washed, purified by recrystallization, and dried.

The resultant 2-hydroxy-3-naphthoic acid is suitably used for various applications, for example, an intermediate for producing colorants, dyes and the like.

The present invention is further described in reference to the following examples. The examples are intended to illustrate the invention and are not to be construed to limit the scope of the invention.

Example 1

Dehydration of hydrous sodium β-naphtholate in the absence of β-naphthol at 290°C

1. Mesuarement of the water content after the dehydration of hydrous sodium β-naphtholate

897 g (2. 7 mole) of 50% by weight of aqueous sodium β-naphtholate was fed in the 1.5-1 separable flask equipped with an agitating screw, a thermometer and a condenser. The solution was heated to 160°C under nitrogen gas flow with stirring to concentrate the solution. Thereafter, 628g of light oil, that had been obtained by distilling off components having boiling point no more than 260°C, was added to the concentrated solution at 160°C. Then, the solution was heated to 290°C, while it was confirmed that sodium β-naphtholate became molten state at a temperature over 260°C.

The mixture of light oil and molten-state sodium β-naphtholate was dehydrated at 290°C with stirring. The stirring was interrupted at 0 min, 30 min, 1 hour and 2 hours after the time when temperature reached to 290°C and at each time point, the mixture was separated into a light oil layer and a sodium β-naphtholate layer. Then, a small amount of the sodium β-naphtholate layer, i.e., the lower layer was sampled and the water content of the sample was measured by the Karl Fischer's method.

2. Measurement of loss of sodium β-naphtholate

The heating of the mixture of light oil and molten-state sodium β-naphtholate was stopped at the time 2 hours later from the time when temperature of the mixture reached to 290°C, and the mixture was cooled to 90°C. After the temperature reached to 90°C, the mixture was transferred to 3-1 glass-made flask having a cock at its bottom. The separable flask used for the dehydration was washed with 2000 g of warm water and the water was added to the latter flask.

Then, the content of glass-made flask was heated to 90°C under nitrogen atmosphere and the temperature was kept constant for 30 minutes with stirring to dissolve the content. Then, the flask was stood so that it was separated into a β-naphthol containing light oil layer and a sodium β-naphtholate containing water layer. The amount of β-naphthol and sodium β-naphtholate contained in each layer was determined with high performance liquid chromatography (HPLC) and the amount of sodium β-naphtholate. recovered after 2-hours dehydration was calculated.

In the HPLC measurement, β-naphthol was used as standard and sodium β-naphtholate was also detected as β-naphthol.

From the above measurement, the loss of sodium β-naphtholate was calculated based on the following formula (I). The result is shown in Table 1.

[Formula (I)]

The loss of sodium β-naphtholate=(C-B)/ (A-D) x100

A: the amount of sodium β-naphtholate fed to the flask (mole)
B: the amount of β-naphthol added upon the dehydration (mole)
C : the amount of sodium β-naphtholate and β-naphthol recovered after the dehydration (mole)
D : the amount of sodium β-naphtholate sampled for the measurement of water content (mole).

In this calculation, all the sampled material was assumed to be sodium β-naphtholate.

Comparative Example 1

Dehydration of hydrous sodium β-naphtholate in the absence of β-naphthol at 250°C

1. Mesuarement of the water content after the dehydration of sodium β-naphtholate

The dehydration of sodium β-naphtholate was performed for 2 hours using the same procedure as Example 1 except that the dehydration temperature was 250°C.

In this example, sodium β-naphtholate did not become molten state, and the mixture of the solvent and sodium β-naphtholate took slurry form. Then the solid-state material was sampled.

2. The measurement of loss of sodium β-naphtholate

After the dehydration was completed, washing water was added to the mixture and then the mixture was separated using the same procedure as Example 1. The amount of recovered sodium β-naphtholate was measured and the loss of sodium β-naphtholate was calculated.

The water content at each sampling time and the loss of sodium β-naphtholate are shown in Table 1.

Comparative Example 2

Dehydration of hydrous sodium β-naphtholate in the presence of 0.5 mole of β-naphthol per 1 mole of sodium β-naphtholate at 260°C

1. Measurement of the water content after the dehydration of sodium β-naphtholate

731 g (2. 2 mole) of 50% by weight of aqueous sodium β-naphtholate was fed in the 1. 5-1 separable flask equipped with an agitating screw, a thermometer and a condenser. The solution was heated to 160°C under nitrogen flow with stirring to concentrate the solution. Thereafter,. 512 g of light oil and 158 g (1. 1 mole) of β-naphthol was added to the concentrated solution at 160°C. Then, the solution was heated to 260°C and at this temperature the dehydration was performed by stirring the mixture of light oil and molten-state sodium β-naphtholate and β-naphthol. The stirring was interrupted at 0 min, 30 min, 1 hour and 2 hours after the time when temperature reached to 260°C and at each time point, the molten-state mixture was separated to a light oil layer and a molten-state mixture layer of sodium β-naphtholate and β-naphthol. Then, a small amount of the sodium β-naphtholate and β-naphthol layer, i.e., the lower layer was sampled and the water content of the sample was measured by the Karl Fischer's method.
In this example, the sample contained both β-naphthol and sodium β-naphtholate and therefore, the content of sodium β-naphtholate in the sample was measured by titration before the measurement of water content. Thereafter, the water content relative to sodium β-naphtholate was calculated.

2. Measurement of the loss of sodium β-naphtholate

After the dehydration was completed, washing water was added to the mixture and then the mixture was separated using the same procedure as Example 1. The amount of recovered sodium β-naphtholate was measured and the loss of sodium β-naphtholate was calculated.
The water content at each sampling time and the loss of sodium β-naphtholate are shown in Table 1.

Table 1

|  | A | B |  | Dehydration time | | | |
| --- | --- | --- | --- | --- | --- | --- | --- |
|  |  |  |  | 0 min | 30 min | 1 h | 2 h |
| Example 1 | 0 | 290 | a | 0.16% | 0.09% | 0.08% | 0.07% |
|  |  |  | b | - | - | - | 1.1% |
| Comparative Example 1 | 0 | 250 | a | 0.42% | 0.41% | 0.39% | 0.36% |
|  |  |  | b | - | - | - | 0.9% |
| Comparative Example 2 | 0.5 | 260 | a | 1.0% | 0.8% | 0.6% | 0.5% |
|  |  |  | b | - | - | - | 5.8% |
| A : Molar amount of β-naphthol per 1 mole of sodium β-naphtholate B:Temperature upon dehydration (°C) a:The water content(% by weight) b : The loss of sodium β-naphtholate (mole %) | | | | | | | |

Example 2

Dehydration of hydrous sodium β-naphtholate in the absence of β-naphthol at 280°C and synthesis of 2-hydroxy-3-naphthoic acid in the presence of β-naphthol

665 g (2. 0 mole) of 50% aqueous sodium β-naphtholate was fed in 2-1 autoclave equipped with an electromagnetic stirring device, a pressure gage and a thermometer. The solution was concentrated by heating to 160°C under nitrogen gas flow with stirring. Thereafter, 465 g of light oil was added slowly to the concentrated solution at 160°C. Then, the solution was heated to 280°C and kept at this temperature for 1 hour with stirring so that sodium β-naphtholate was dehydrated.
After the dehydration was completed, 144g (1. 0 mole) of β-naphthol was added to the dehydrated sodium β-naphtholate. The resultant mixture was heated to 290°C and at this temperature, the mixture was reacted with carbon dioxide under a pressure of 0. 6 MPa (G) for 1.8 hours. After the reaction, the autoclave was cooled to 90°C and 2000 g of water was added to the reaction. The resultant mixture was stirred under nitrogen gas flow at 90°C for 30 min so that the content was dissolved. To the resultant solution, 500 g of xylene was added and the mixture was separated at 90°C into a solvent layer containing light oil and xylene (upper layer) and an aqueous layer containing sodium 2-hydroxy-3-naphtholate (lower layer).

The content of each layer was analyzed with HPLC and the amount of the starting material and the reaction product was measured. The result is shown in Table 2. From this analysis, the yield of 2-hydroxy-3-naphthoic acid based on the amount of sodium β-naphtholate was found to be 40. 0% and the yield of side-products was found to be 7. 1 %.

2600 g of the above-described aqueous layer (contained 166 g of mono-sodium 2-hydroxy-3-naphthoate) was adjusted to pH 6.7 using 73 % sulfuric acid at 90°C. Then at this temperature, the fraction was extracted three times with 400 g of xylene. After the extraction with xylene, the aqueous layer was adjusted to pH 2.5 using 73 % sulfuric acid at 90°C. This acid crystallization step gave 2-hydroxy-3-naphthoic acid suspension. The suspension was centrifuged, washed and dried to give the pale yellow crystal of 2-hydroxy-3-naphthoic acid (143 g).

The ML value of the obtained crystal was measured according to the following method. The ML value provides an indication of coloring due to the tar-like side-product. The result is shown in Table 2.

[Measurement of ML value]

2-hydroxy-3-naphthoic acid is dissolved in methanol and optical density at 530nm (OD 530) of the solution containing 1g of 2-hydroxy-3-naphthoic acid is measured. The ML value is the value 200 times of the OD 530. The higher the ML value is, the more the product is colored by the tar-like substance.

6g of the product was sampled and dissolved in methanol so that total volume is 200 ml. After the solution is filtrated with No.5A filter paper (φ 12. 5cm), OD 530 was measured. Methanol was used as standard. Then, ML value was calculated as follows:

$$ML\ value = OD\ 530 / (amount\ of\ the\ sample\ (g)) \times 200$$

Example 3

Dehydration of hydrous sodium β-naphtholate in the absence of β-naphthol at 280°C and synthesis of 2-hydroxy-3-naphthoic acid in the absence of β-naphthol

665 g (2. 0 mole) of 50% aqueous sodium β-naphtholate was fed in 2-1 autoclave equipped with an electromagnetic stirring device, a pressure gage and a thermometer. The solution was concentrated by heating to 160°C under nitrogen gas flow with stirring. Thereafter, 465 g of white oil (liquid paraffin) was added slowly to the concentrated solution at 160°C. Then, the solution was heated to 280°C and kept at this temperature for 1 hour with stirring so that sodium β-naphtholate was dehydrated.

After the dehydration was completed, the resultant mixture was heated to 290°C and at this temperature, the mixture was reacted with carbon dioxide under a pressure of 0. 6 MPa (G) for 1. 8 hours. After the reaction, the autoclave was cooled to 90°C and 2000 g of water was added to the reaction. The resultant mixture was stirred under nitrogen gas flow at 90°C for 30 min so that the content was dissolved. To the resultant solution, 500 g of xylene was added and the mixture was separated at 90°C into a solvent layer containing white oil and xylene (upper layer) and an aqueous layer containing sodium 2-hydroxy-3-naphtholate (lower layer).

The content of each layer was analyzed with HPLC and the amount of the starting material and reaction. product was measured. The result is shown in Table 2. From this analysis, the yield of 2-hydroxy-3-naphthoic acid based on the amount of sodium β-naphtholate was found to be 36. 7% and the yield of side-products was found to be 7. 6%.

2600 g of the above-described aqueous layer (contained 153 g of mono-sodium 2-hydroxy-3-naphtholate) was batched off. The fraction was treated by the same procedure as that of Example 2 to give 131 g of pale yellow crystal of 2-hydroxy-3-naphthoic acid. The ML value of the crystal is shown in table 2.

Comparative Example 3

Dehydration of hydrous sodium β-naphtholate in the presence of 0. 5 mole of β-naphthol per 1 mole of sodium β-naphtholate at 260°C and synthesis of 2-hydroxy-3-naphthoic acid in the presence of β-naphthol

665 g (2.0 mole) of 50% aqueous sodium β-naphtholate was fed in 2-1 autoclave equipped with an electromagnetic stirring device, a pressure gage and a thermometer. The solution was concentrated by heating to 160°C under nitrogen gas flow with stirring. Thereafter, 144 g (1. 0 mole) of β-naphthol was added to the solution at 160°C and then, 465 g of light oil was added slowly to the solution. Then, the solution was heated to 260°C and kept at this temperature for 2 hours with stirring so that sodium β-naphtholate was dehydrated.

After the dehydration was completed, the resultant mixture was heated to 290°C and at this temperature, the mixture was reacted with carbon dioxide under a pressure of 0. 6 MPa (G) for 1. 8 hours. After the reaction, the autoclave was cooled to 90°C and 2000 g of water was added to the reaction. The resultant mixture was stirred under nitrogen gas flow at 90°C for 30 min so that the content was dissolved. To the resultant solution, 500 g of xylene was added and the mixture was separated at 90°C into a solvent layer containing light oil and xylene (upper layer) and an aqueous layer containing sodium 2-hydroxy-3-naphtholate (lower layer).

The content of each layer was analyzed with HPLC and the amount of the starting material and the reaction product was measured. The result is shown in Table 2. From this analysis, the yield of 2-hydroxy-3-naphthoic acid based on the amount of sodium β-naphtholate was found to be 37. 0 % and the yield of side-products was found to be 14. 2 %.

2600 g of the above-described aqueous layer (contained 154 g of mono-sodium 2-hydroxy-3-naphtholate) was batched off. The fraction was treated by the same procedure as that of Example 2 to give 132 g of pale yellow crystal of 2-hydroxy-3-naphthoic acid. The ML value of the crystal is shown in Table 2.

Table 2.

| | Upper raw: yield (mole %) based on the amount of sodium β-naphtholate Lower raw: recovered amount (mole) of the product | | | | | ML value |
|---|---|---|---|---|---|---|
| | A | B | C | D | E | |
| Example 2 | 40.0 | 0.2 | 0.03 | 52.7 | 7.1 | 0.34 |
| | 0.80 | 0.004 | 0.001 | 1.05 | 0.14 | |
| Example 3 | 36.7 | 0.9 | 0.05 | 54.8 | 7.6 | 0.30 |
| | 0.73 | 0.018 | 0.001 | 1.10 | 0.15 | |
| Comparative Example 3 | 37.0 | 0.3 | 0.05 | 48.8 | 14.2 | 0.55 |
| | 0.74 | 0.006 | 0.001 | 0.98 | 0.27 | |

A:2-hydroxy-3-naphthoic acid

B:2-hydroxy-6-naphthoic acid

C:2-hydroxy naphthalene 3, 6-dicarboxylic acid

D : sodium β-naphtholate

(Total amount (mole) of recovered β-naphthol and sodium β-naphtholate)-(total amount (mole) of β-naphthol added upon the dehydration and/or the reaction )

E : side-products

Yield: (100 (%))-(total yield (mole %) of A to D);

Recovered amount: (the amount (moles) of fed sodium β-naphtholate)-(total recovered amount (moles) of A to D)

## Claims

1. A process for dehydrating hydrous sodium β-naphtholate comprising:

   the step of heating the hydrous sodium β-naphtholate or a mixture of the hydrous sodium β-naphtholate and β-naphthol which comprises no more than 0.2 mole of β-naphthol per one mole of sodium β-naphtholate in a solvent at a temperature of 260-300°C under an inert gas.

2. The process of Claim 1, wherein the solvent is selected from the group consisting of light oil, kerosene, white oil and mixture thereof.

3. The process of Claim 1, wherein the hydrous sodium β-naphtholate or the mixture of the hydrous sodium β-naphtholate and β-naphthol in a solvent is heated for 0.5-2.0 hours.

4. A process for manufacturing 2-hydroxy-3-naphthoic acid, comprising the steps of:

heating the hydrous sodium β-naphtholate or a mixture of the hydrous sodium β-naphtholate and β-naphthol which comprises no more than 0.2 mole of β-naphthol per one mole of sodium β-naphtholate in a solvent at a temperature of 260-300°C under an inert gas to dehydrate the hydrous sodium β-naphtholate; and reacting the dehydrated sodium β-naphtholate in the solvent with carbon dioxide.

5. The process of Claim 4, wherein the solvent is selected from the group consisting of light oil, kerosene, white oil and mixture thereof.

6. The process of Claim 4, wherein the hydrous sodium β-naphtholate or the mixture of the hydrous sodium β-naphtholate and β-naphthol in a solvent is heated for 0.5-2.0 hours.

7. The process of Claim 4, further comprising the step of adding β-naphthol to the dehydrated sodium β-naphtholate in the solvent.

8. The process of claim 7, wherein the amount of β-naphthol added to the dehydrated sodium β-naphtholate is 0.1-3.0 mole per one mole of sodium β-naphtholate.

**European Patent Office**

**EUROPEAN SEARCH REPORT**

Application Number

EP 04 01 9324

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | GB 1 443 987 A (BASF AG) 28 July 1976 (1976-07-28) * page 7, lines 7-10; examples 1-2, 4-10 * ----- | 1,3,4,6 | C07C37/70 C07C39/235 C07C51/15 C07C65/11 |
| Y,D | US 4 239 913 A (R. UENO ET AL) 16 December 1980 (1980-12-16) * the whole document, in particular column 4, lines 30-57, examples 4, 6 * ----- | 1-8 | |
| Y | EP 0 327 221 A (KK UENO SEIYAKU) 9 August 1989 (1989-08-09) * page 4, lines 6-14; example 1 * ----- | 1-8 | |
| A | GB 1 464 418 A (BASF AG) 16 February 1977 (1977-02-16) * examples 3-4 * ----- | 1,3,4,6 | |
| A | US 1 503 984 A (L.H. CONE) 5 August 1924 (1924-08-05) * the whole document * ----- | 1,4 | |

TECHNICAL FIELDS SEARCHED (Int.Cl.7)

C07C

The present search report has been drawn up for all claims

| Place of search | Date of completion of the search | Examiner |
|---|---|---|
| Berlin | 22 October 2004 | Van Amsterdam, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 1 506 950 A1

## ANNEX TO THE EUROPEAN SEARCH REPORT
## ON EUROPEAN PATENT APPLICATION NO.

EP 04 01 9324

This annex lists the patent family members relating to the patent documents cited in the above-mentioned European search report.
The members are as contained in the European Patent Office EDP file on
The European Patent Office is in no way liable for these particulars which are merely given for the purpose of information.

22-10-2004

| Patent document cited in search report | | Publication date | Patent family member(s) | | Publication date |
|---|---|---|---|---|---|
| GB 1443987 | A | 28-07-1976 | DE | 2260637 A1 | 20-06-1974 |
| | | | DE | 2328156 A1 | 19-12-1974 |
| | | | BE | 808540 A1 | 12-06-1974 |
| | | | CH | 605569 A5 | 29-09-1978 |
| | | | FR | 2209741 A1 | 05-07-1974 |
| | | | IT | 1002180 B | 20-05-1976 |
| | | | JP | 49087652 A | 22-08-1974 |
| | | | NL | 7317030 A | 14-06-1974 |
| | | | US | 4038309 A | 26-07-1977 |
| US 4239913 | A | 16-12-1980 | JP | 1183488 C | 27-12-1983 |
| | | | JP | 54079256 A | 25-06-1979 |
| | | | JP | 58001098 B | 10-01-1983 |
| | | | JP | 1112175 C | 16-09-1982 |
| | | | JP | 54079257 A | 25-06-1979 |
| | | | JP | 56053296 B | 17-12-1981 |
| | | | CH | 643526 A5 | 15-06-1984 |
| | | | DE | 2837053 A1 | 26-04-1979 |
| | | | FR | 2407196 A1 | 25-05-1979 |
| | | | GB | 2008090 A ,B | 31-05-1979 |
| | | | IT | 1111198 B | 13-01-1986 |
| | | | NL | 7809824 A ,B, | 27-04-1979 |
| EP 0327221 | A | 09-08-1989 | JP | 1190649 A | 31-07-1989 |
| | | | JP | 2718932 B2 | 25-02-1998 |
| | | | CN | 1034362 A ,B | 02-08-1989 |
| | | | DE | 68905625 D1 | 06-05-1993 |
| | | | DE | 68905625 T2 | 08-07-1993 |
| | | | EP | 0327221 A1 | 09-08-1989 |
| | | | ES | 2055028 T3 | 16-08-1994 |
| | | | IL | 88929 A | 31-07-1994 |
| | | | KR | 121289 B1 | 13-11-1997 |
| | | | US | 4966992 A | 30-10-1990 |
| GB 1464418 | A | 16-02-1977 | DE | 2332064 A1 | 16-01-1975 |
| | | | BE | 816677 A1 | 23-12-1974 |
| | | | CH | 588436 A5 | 31-05-1977 |
| | | | DD | 111898 A5 | 12-03-1975 |
| | | | FR | 2234266 A1 | 17-01-1975 |
| | | | IT | 1014812 B | 30-04-1977 |
| | | | JP | 50036449 A | 05-04-1975 |
| | | | NL | 7408157 A | 30-12-1974 |
| | | | SU | 548203 A3 | 25-02-1977 |
| | | | US | 4032568 A | 28-06-1977 |
| US 1503984 | A | 05-08-1924 | NONE | | |

EPO FORM P0459

For more details about this annex : see Official Journal of the European Patent Office, No. 12/82

11